# EUROPÄISCHE PATENTANMELDUNG

(11) **EP 4 480 517 A1**
(43) Veröffentlichungstag der Anmeldung: **25.12.2024**
(21) Anmeldenummer: 23180471.7
(22) Anmeldetag: 20.06.2023
(51) Int. Cl.: A61M 5/20

(54) **AUTOINJEKTOR ZUR INTRAVITREALEN INJEKTION**

(71) Anmelder: Ypsomed AG, 3401 Burgdorf (CH)
(72) Erfinder: Schenker, Susanne, 4912 Aarwangen (CH); von Mühlenen, Beat, 3043 Uetligen (CH)
(74) Vertreter: Meier Obertüfer, Jürg

(57) **Zusammenfassung**

Die Erfindung betrifft einen Autoinjektor (1) zur Verabreichung eines Medikaments umfassend: - ein einteiliges oder mehrteiliges Gehäuse (10,20) mit einer Längsachse (L) und einer gehäusefest koaxial aufgenommenen Spritze (30) mit einem verschiebbaren Kolben (30c) - eine distal am Gehäuse (10,20) angebrachte Aufnahme (10g, 10h) oder einen koaxialer Durchgang für eine Nadel (110) - einen proximal am Gehäuse (10,20) angebrachten von Hand schraubbaren Knopf (40) - eine axial wirkende erste Feder (90) welche vorgespannt im Knopf (40) koaxial gelagert ist - eine am Knopf (40) befestigte und von diesem lösbare Kolbenstange (70) welche von der ersten Feder (90) in distaler Richtung kraftbeaufschlagt ist - ein Auslöseelement (50) welches in einer ersten Lage die Kolbenstange (70) axial gegen die Kraft der ersten Feder (90) zum schraubbaren Knopf (40) befestigt hält und welches Auslöseelement (50) in einer zweiten Lage die Kolbenstange (70) vom Knopf (40) lösbar macht wodurch der verschiebbare Kolben (30c) in der Spritze (30) von der Kraft der ersten Feder (90) mittels der gelösten Kolbenstange (70) in distale Richtung um einen Kolbenweg verschoben werden kann wobei das Auslöseelement (50) für eine Bewegung in die zweite Lage kraftbeaufschlagbar wird, während der Knopf (40) und die daran befestigte Kolbenstange (70) aus einer axialen Anfangsposition relativ zum Gehäuse (10,20) durch Einschrauben ins Gehäuse (10,20) eine axiale Primingposition erreichen, wobei die Kolbenstange (70) den Kolben (30c) in der Spritze (30) in distale Richtung stösst.

## Beschreibung

### TECHNISCHES GEBIET

Die vorliegende Erfindung betrifft das Gebiet der medizinischen Injektionsgeräte zur Verabreichung von flüssigen Substanzen, insbesondere von Medikamenten oder medizinischen Substanzen. Die Erfindung bezieht sich auf einen Autoinjektor mit einem Energiespeicher zur Ausschüttung einer vorgegebenen Dosis aus einem einmalig genutzten Produktbehälter gemäss dem Oberbegriff von Anspruch 1. Der erfindungsgemässe Autoinjektor ist zur Injektion in das Innere eines Auges, insbesondere in den Glaskörper des Auges geeignet aber auch für Injektionen in andere Teile des Auges oder in andere menschliche oder tierische Körperteile oder Organe bzw. Gewebe.

Die Fähigkeit, Medikamente über eine intravitreale Injektionstherapie (IVT) direkt in das Auge zu verabreichen, hat die Behandlungslandschaft einer Reihe von zuvor zu Erblindung führenden Krankheiten, einschließlich Makuladegeneration und diabetischer Retinopathie, verändert. Der Erfolg dieser Therapien bei der Verhinderung von Erblindung hat zu einem dramatischen Anstieg der Zahl der durchgeführten intravitrealen Injektionen geführt. Die Zahl der Indikationen für IVT nimmt weiter zu, wodurch die Inanspruchnahme dieser Therapie jedes Jahr deutlich zunimmt. Die primären Einschränkungen der IVT sind Patientenbeschwerden, Blutungen an der Augenoberfläche, Hornhauttoxizität und die zeitlichen Einschränkungen bei der Behandlung der großen Anzahl von Patienten, die diese Therapie benötigen.

### HINTERGRUND DER ERFINDUNG

DE102005022532 A1 (Hommann) zeigt eine Verabreichungsvorrichtung zur Verabreichung eines Produktes mit vorhergehender Durchführung eines Priming-Vorganges, wobei die Vorrichtung ein Gehäuse zur Aufnahme einer Ampulle mit dem Produkt und einer Mechanik zum Vortrieb eines Stopfens in der Ampulle aufweist. Die Anordnung enthält eine Priming-Stange, die durch Betätigung des Priming-Knopfes auf den Stopfen einwirkt. Während dabei der Priming-Vorgang durchgeführt wird, wird hierdurch die Mechanik zum Vortrieb des Stopfens zur Verabreichung des Produktes ebenfalls freigegeben. Dabei weist die Mechanik eine Verabreichungsstange auf, die in Folge der Betätigung eines Verabreichungsknopfes den Stopfen innerhalb der Ampulle vorantreibt, um eine Verabreichung des Produktes durchzuführen. Die Vorrichtung ist nicht geeignet für eine Injektion ins Auge und die komplexe Mechanik mit zwei Stangen kompromittiert die Präzision der Dosierung des zu verabreichenden Produkts, da der Priming-Vorgang vorhandenes Spiel und Toleranzen in der Mechanik zum Vortrieb des Stopfens zur Verabreichung nicht aufhebt. Weiter ist von aussen nicht deutlich erkennbar, ob ein Priming durchgeführt ist.

US2008097390A1 (Alcon) zeigt ein federbetätigtes Abgabesystem mit einem Dosierelement, einem Kolben, einer Feder, einer Welle und einem Verriegelungsmechanismus. Das Dosierelement verfügt über eine Dosierspitze und eine Kanüle. Der Kolben ist mit der Innenfläche des Dosierelements verbunden und kann in den Hohlraum des Dosierelements gleiten. Der Kolben ist fließend mit der Innenfläche des Dosierelements abgedichtet. Die Feder ist über die Welle mit dem Kolben verbunden. Der Verriegelungsmechanismus greift in die Welle ein und hält sie in einer ersten Position, so dass sich die Feder in einem zusammengedrückten Zustand befindet. Wenn der Verriegelungsmechanismus losgelassen wird, fährt die Feder aus und treibt so die Welle und den angeschlossenen Kolben in Richtung des sich verjüngenden Endes des Dosierelements an. Der Kolben stösst die Substanz aus dem Abgabeelement in das Auge aus. Das Abgabesystem erlaubt kein Priming und signalisiert das Ende der Substanzausstossung nicht.

US2010241102A1 (Colin) zeigt eine Injektionsanordnung, die zur Aufnahme eines Injektors angepasst ist, und eine Ausrichtungsführung, die angepasst ist, um die Platzierung des Injektors relativ zum Auge eines Patienten zu erleichtern, um einen Substanz zu injizieren. Die Injektionsbaugruppe wird als Reaktion auf eine Benutzereingabe so angepasst, dass sie zuerst automatisch und sequentiell den Injektor in das Auge des Patienten einschiebt und dann die Substanz aus dem Injektor abgibt. Das Injektionsanordnung erlaubt kein Priming und signalisiert das Ende der Substanzausstossung nicht.

WO14005728A1 (Novartis) zeigt eine vorgefüllte Spritze, wobei die Spritze einen Körper, einen Stopfen und einen Kolben umfasst, wobei der Körper einen Auslass an einem Auslassende umfasst und der Stopfen innerhalb des Körpers so angeordnet ist, dass eine Vorderseite des Stopfens und des Körpers eine Kammer mit variablem Volumen definieren, aus der eine Flüssigkeit durch den Auslass ausgestoßen werden kann, wobei die Flüssigkeit eine ophthalmologische Substanz beinhaltet.

WO14179698A2 (Clearside) zeigt eine Vorrichtung mit einem Gehäuse, das so konfiguriert ist, dass es mit einem Medikamentenbehälter gekoppelt ist. Der Medikamentenbehälter ist so konfiguriert, dass er mit einer Nadel gekoppelt ist. Eine Einspritzbaugruppe ist innerhalb des Gehäuses angeordnet und umfasst ein Energiespeicherelement und eine Betätigungsstange. Ein distaler Endabschnitt der Betätigungsstange ist so konfiguriert, dass er innerhalb des Arzneimittelbehälters angeordnet werden kann. Das Energiespeicherelement ist so konfiguriert, dass es eine Kraft auf einen proximalen Endabschnitt der Betätigungsstange erzeugt. Die Kraft reicht aus, um den distalen Endabschnitt der Betätigungsstange innerhalb des Arzneimittelbehälters zu bewegen, um zumindest einen Teil einer Substanz aus dem Arzneimittelbehälter über die Nadel zu befördern. Ein proximaler Endabschnitt eines Einstellelements ist so konfiguriert, dass er mit dem Medikamentenbehälter gekoppelt ist. Ein distaler Endabschnitt des Einstellelements ist mit einer Nadel gekoppelt. Das Verstellelement ist innerhalb des Gehäuses beweglich angeordnet, so dass, wenn das Verstellelement relativ zum Gehäuse gedreht wird, die Nadel durch eine Vielzahl von diskreten Schritten entlang einer Längsachse des Gehäuses bewegt wird.

WO19202603A1 (Everads) zeigt eine Vorrichtung zur Injektion in eine Zwischenschicht eines Organs eines Subjekts. Die Vorrichtung enthält eine Nadel mit einem Größenübergangsabschnitt, der in einer scharfen Nadel-Distalspitze endet. Das Gerät enthält auch einen länglichen Gewebeseparator, der im Nadellumen verschiebbar ist. Ein Aktuator verschiebt den Gewebeseparator im Nadellumen zwischen einer ersten Position, in der die distale Spitze des Separators proximal zur distalen Nadelspitze positioniert ist, und einer zweiten Position, in der die distale Spitze des Separators in einem vorgegebenen Abstand distal von der distalen Nadelspitze fest positioniert ist.

WO20009892A1 (Irenix) zeigt Vorrichtungen und Verfahren zur intravitrealen Verabreichung. Ein Wirkstoffverabreichungsmodul ist zur Aufnahme einer vorgefüllten Spritze konfiguriert und kann mit einer Nadeleinheit gekoppelt werden. Das Wirkstoffverabreichungsmodul ist mit einem Aktuator verbindbar, welcher automatisches Priming durchführt, die Nadel in das Auge einführt und schliesslich den Kolben in der Spritze derart bewegt, dass eine gewünschte Dosis eines Wirkstoffs durch die Nadel in das Auge abgegeben wird. Nach Gebrauch kann das Wirkstoffverabreichungsmodul vom Aktuator getrennt werden und der Aktuator kann in eine Dockingstation platziert werden.

WO20212476A1 (Böhringer) zeigt verschiedene Ausführungsformen einer Verabreichungsvorrichtung für die intravitreale Injektion eines Wirkstoffes aus einer in der Verabreichungsvorrichtung enthaltenen Spritze und einem Primingmechanismus mit einer Primingkolbenstange. Die Verabreichung wird unabhängig vom Primingmechanismus über einen manuell betätigten Mechanismus bewirkt, welcher die Spritze mit der Nadel und eine Verabreichungskolbenstange mittels eines Getriebes bewegen kann. Die manuelle Betätigung kann durch radiales Eindrücken eines seitlichen Knopfs oder durch axiale Relativverschiebung von Gehäuseteilen erfolgen. Mittels einer Rückholfeder ist die Spritze mit der Nadel wieder in eine Ausgangsposition bringbar.

Trotz diesen Bemühungen besteht nach wie vor Bedarf an einem zuverlässigen, sicheren und kostengünstigen System zur zeitsparenden Injektion präziser Substanzvolumina in das Auge oder andere Organe oder Gewebe.

### DARSTELLUNG DER ERFINDUNG

Es ist eine Aufgabe der Erfindung, einen Autoinjektor der eingangs genannten Art zu schaffen, welcher sicher und einfach anwendbar ist. Es soll ein vollständiges Primen erfolgen und eine exakte Dosierung des zu verabreichenden Produkts gewährleistet sein. Die Bedienung und Handhabung des Autoinjektors soll Irritation an der Verabreichungsstelle minimieren und die unterschiedlichen Gebrauchszustände des Autoinjektors sollen für den Anwender sicher erkennbar bzw. wahrnehmbar sein.

Die Aufgabe wird gelöst durch Vorrichtungen mit den Merkmalen des unabhängigen Anspruchs. Bevorzugte Ausführungsformen der Erfindung sind Gegenstand der abhängigen Ansprüche.

Ein erfindungsgemässer Autoinjektor zur Verabreichung eines Medikaments umfasst:
- ein einteiliges oder mehrteiliges Gehäuse mit einer Längsachse und einer gehäusefest koaxial aufgenommenen Spritze mit einem verschiebbaren Kolben
- eine distal am Gehäuse angebrachte Aufnahme für eine Nadel
- einen proximal am Gehäuse angebrachten von Hand schraubbaren Knopf
- eine erste axial wirkende Feder welche vorgespannt im Knopf koaxial gelagert ist
- eine am Knopf befestigte und von diesem lösbare Kolbenstange welche von der ersten Feder in distaler Richtung kraftbeaufschlagt ist
- ein Auslöseelement welches in einer ersten axialen Lage die Kolbenstange axial gegen die Kraft der ersten Feder zum schraubbaren Knopf befestigt hält und welches Auslöseelement in einer zweiten axialen Lage die Kolbenstange vom Knopf lösbar macht wodurch der verschiebbare Kolben in der Spritze von der Kraft der ersten Feder mittels der gelösten Kolbenstange in distale Richtung um einen Kolbenweg verschoben werden kann. Das Auslöseelement wird für eine Bewegung in die zweite Lage kraftbeaufschlagbar oder kraftbeaufschlagt während der Knopf und die daran befestigte Kolbenstange aus einer axialen Anfangsposition relativ zum Gehäuse durch Einschrauben ins Gehäuse eine axiale Primingposition Anfangsposition relativ zum Gehäuse erreichen, wobei die Kolbenstange den Kolben in der Spritze in distale Richtung stösst.

Durch die Bewegung der am Knopf befestigten Kolbenstange aus einer axialen Anfangsposition relativ zum Gehäuse in eine axiale Primingposition wird der Kolben in der Spritze verschoben was bewirkt, dass die verdrängte Flüssigkeitsmenge Luft aus dem Produktbehälter und aus dem Lumen der angeschlossenen Nadel verdrängt, wobei ein Teil der Flüssigkeitsmenge aus dem distalen Ende der Nadel in die Umgebung austreten kann. Der Kolben in der Spritze wird bis zum Erreichen der axialen Primingposition so weit in distaler Richtung vorgeschoben, dass das in der Spritze verbleibende verdrängbare Volumen einer bestimmten zu verabreichenden Dosis entspricht. Die Präzision der Dosis ist dadurch gewährleistet, indem bei diesem (Priming-) Vorgang alle auch an der nachfolgenden Ausschüttung beteiligten Elemente wie Spritze, Kolben, Kolbenstange, erste Feder, Auslöseelement etc. zusammen und gleichzeitig mitbewegt bzw. belastet werden und damit allenfalls vorhandenes präzisionskompromittierendes Spiel und elastizitätsbedingte Ausgleichsbewegungen aufgehoben werden.

Durch die Bewegung der gelösten Kolbenstange in distale Richtung um den Kolbenweg aus der axialen Primingposition wird der Kolben in der Spritze um den Kolbenweg verschoben was bewirkt, dass ein definiertes Volumen bzw. eine bestimmte Dosis Medikament aus der Spritze verdrängt bzw. durch die Nadel ausgeschüttet wird.

Bevorzugt kann der Knopf in seiner axialen Anfangsposition bereits teilweise in das Gehäuse eingeschraubt sein und seine axiale Primingposition durch weiteres Einschrauben erreichen.

Bevorzugt stösst an der axialen Primingposition ein radial gerichteter Anschlagnocken am Knopf an eine radial gerichtete Anschlagrippe am Gehäuse. Dadurch wird ein präziser Halt des Einschraubens bewirkt.

Bevorzugt ist der Autoinjektor weitergebildet indem:
- eine zweite axial wirkende Feder welche mit ihrem distalen Ende am proximalen Flansch der Spritze und mit ihrem proximalen Ende an einem distalen Nocken am Knopf gelagert ist, wobei ein elastischer Abschnitt der zweiten Feder durch das Einschrauben des Knopfs ins Gehäuse gespannt wird und vor dem Erreichen der axialen Primingposition das proximale Ende der zweiten Feder vom distalen Nocken am Knopf kinematisch auf das Auslöseelement wechselt und dieses kraftbeaufschlagt indem das proximale Ende der zweiten Feder das Auslöseelement in proximale Richtung drängt, insbesondere indem durch das Einschrauben des Knopfs ins Gehäuse ein Haltenocken der zweiten Feder in eine Halteöffnung in einer axial verlaufenden Rille des Auslöseelements einkuppelt und der Nocken am Knopf durch einen axialen Ausschnitt am Endabschnitt der zweiten Feder freigestellt wird.

Durch die so gespannte oder weitergespannte Feder wird die Spritze sicher und spielfrei zum Gehäuse fixiert. Die beteiligten Elemente bleiben vor dem Spannen der zweiten Feder kraftfrei und sind damit als Kunststoffteile während einer Lagerzeit bzw. vor dem Gebrauch geschützt vor Formänderung durch Relaxation. Eine Betätigung des Autoinjektors mittels einem Betätigungselement bleibt zudem vor einer Aktivierung des Auslöseelements durch die Kraftbeaufschlagung wirkungslos.

Der Drehwinkel der Einschraubbewegung welcher den Knopf und die daran befestigten Teile relativ zum Gehäuse aus ihrer axialen Anfangsposition in die axiale Primingposition bringt ist vorzugsweise kleiner als 360°, bevorzugter kleiner als 180°, noch bevorzugter 165° bis 75°, insbesondere 120°. Dadurch ist der Primingvorgang bzw. die Aktivierung ergonomisch ohne Nachgreifen von Hand ausführbar. Bevorzugt stösst beim Erreichen der axialen Primingposition bzw. am Ende des Drehwinkels ein radial gerichteter Anschlagnocken am Knopf an eine radial gerichtete Anschlagrippe am Gehäuse. Dadurch wird ein präziser Halt des Einschraubens und damit ein präzises Ende des Primings bzw. ein präziser Anfang des Kolbenwegs für die nachfolgende Injektion bewirkt.

Bevorzugt ist der Autoinjektor weitergebildet, indem ein elastischer Abschnitt der zweiten Feder aus einem Kunststoff gebildet ist. Dadurch kann die zweite Feder kostengünstig insbesondere einteilig im Kunststoffspritzgussverfahren hergestellt werden. Die Montage des Autoinjektors ist einfacher durchzuführen, da die zweite Feder beim Einbau ungespannt oder nur leicht vorgespannt bleibt. Alternativ kann die zweite Feder auch kombiniert aus einer Metallfeder und Kunststoffteilen gebildet sein.

Bevorzugt ist der Autoinjektor weitergebildet, indem der proximal in das Gehäuse von Hand schraubbare Knopf und das Gehäuse am äusseren Umfang je eine Markierung aufweisen, welche in der axialen Primingposition des Knopfs aufeinander weisen, insbesondere in achsparalleler Linie zu stehen kommen. Dadurch wird sicher erkennbar, dass der Autoinjektor geprimt und bereit zur Injektion ist.

Bevorzugt ist der Autoinjektor weitergebildet, indem der proximal in das Gehäuse von Hand schraubbare Knopf oder das Gehäuse einen Schnapphaken aufweist, welcher in der axialen Primingposition den Knopf zum Gehäuse rotativ und/oder axial fixiert. Dadurch ist verhindert, dass der Knopf nach dem Primen wieder zurückgeschraubt werden kann. Weiter signalisiert das dabei entstehende Klickgeräusch das vollständige Primen.

Bevorzugt ist der Autoinjektor weitergebildet indem:
- proximal vom Auslöseelement ein Sperrring gebildet ist welcher vom Auslöseelement bei der Bewegung von der ersten Lage in die zweite Lage in proximale Richtung bewegbar ist, wobei der Sperrring in der ersten Lage des Auslöseelements einen an der Kolbenstange gebildeten Sperrarm am radialen Auslenken hindert und im Eingriff mit einer Haltekante am Knopf hält und/oder der Sperrring in der zweiten Lage des Auslöseelements den an der Kolbenstange gebildeten Sperrarm für eine radiale Auslenkung zur Längsachse hin freigibt, wodurch die Kolbenstange vom Knopf lösbar wird. Dadurch kann die Kraft der ersten Feder den Sperrarm an der Kolbenstange radial auslenken und die Kolbenstange relativ zum Gehäuse in distale Richtung bewegen.

Alternativ kann der Sperrarm auch am Knopf und die Haltekante an der Kolbenstange gebildet sein.

Bevorzugt ist der Sperring gegenüber der Kolbenstange durch einen Kraftschluss axial gehalten, insbesondere durch einen flexiblen Haltearm am Sperrring, welcher distal hinter eine Haltekante an der Kolbenstange greift. Bei der Bewegung des Auslöseelement von der ersten Lage in die zweite Lage in proximale Richtung wird der Kraftschluss am mitbewegten Sperring gelöst, indem der Haltearm elastisch über die Haltekante gleiten kann. Bevorzugt ist der Autoinjektor weitergebildet indem:
- ein Betätigungselement insbesondere seitlich am Gehäuse gebildet ist wobei das Betätigungselement in seiner unbetätigten Position das Auslöseelement in der ersten Lage hält und/oder das Betätigungselement in seiner betätigten Position das Auslöseelement für eine Bewegung in die zweite Lage freigibt. Dadurch kann die Kraft der gespannten zweiten Feder das Auslöseelement in proximale Richtung axial bewegen wodurch dieses von der ersten in die zweite Lage bewegt wird. Das Betätigungselement kann seitlich am Gehäuse insbesondere in einem distalen Abschnitt des Gehäuses als axial und/oder tangential verschiebbares oder als radial eindrückbares Betätigungselement als dreh- oder schwenkbare Wippe oder als Schieber oder in knopfform ausgestaltet sein.

Bevorzugt ist der Autoinjektor weitergebildet, indem der Kolben von der Kraft der ersten Feder mittels der gelösten Kolbenstange in distale Richtung um den Kolbenweg verschiebbar ist an dessen Ende die Kolbenstange mit einem Stoppnocken an einer Stoppkante am Gehäuse auftrifft, wobei das durch den Kolbenweg aus der Spritze verdrängbare Volumen aus dem Bereich 0.01 bis 0.5 ml, bevorzugter 0.02 bis 0.1 ml, noch bevorzugter 0.04 bis 0.06 ml, insbesondere 0.05 ml ist. Durch die durch massgenau herstellbare Kunsttoffteile definierten Stoppelemente wird das verdrängbare Volumen präziser gegenüber einer Ausschüttung, welche durch blosses Anstossen des Kolbens aus Gummi am Boden der Spritze aus Glas beendet wird.

Bevorzugt ist der Autoinjektor weitergebildet, indem:
- eine koaxial im Knopf gelagerte durch die proximal wirkende Kraft der ersten Feder um einen Hub aus einer Ausgangslage verschiebbaren Signalhülse gebildet ist, wobei die Signalhülse am Ende des Hubs mittels eines Anschlagflansches auf einen Anschlagnocken am Knopf auftrifft und/oder ein verjüngter Endabschnitt der Signalhülse am proximalen Ende des Knopfes exponiert wird. Dadurch entsteht ein Auftreffgeräusch, welches hör- und/oder spürbar das Ende der Injektion angibt sowie eine sichtbare Anzeige, welche persistent und deutlich den Autoinjektor als gebraucht ausweist.

Bevorzugt ist der Autoinjektor weitergebildet, indem die proximale Stirnseite der Rippe an der Kolbenstange das distale Ende der ersten Feder lagert und/oder der Flansch der Signalhülse das proximale Ende der ersten Feder lagert.

Bevorzugt ist der Autoinjektor weitergebildet, indem die Signalhülse in der Ausgangslage über einen durch Auslenkung lösbaren Schnapparm an einer Haltekante am Knopf befestigt ist, wobei der Schnapparm von der Aussenseite einer Rippe an der Kolbenstange bis vor dem Ende des Kolbenhubs an der Auslenkung gehindert wird. Dadurch wir der Hub und damit die Signalisierung erst unmittelbar vor dem Ende der Injektion ausgelöst. Weiter bevorzugt wird die Signalhülse am Ende des Hubs zum Knopf axial verriegelt, wodurch der verjüngte Endabschnitt der Signalhülse nicht mehr in den Knopf zurückgeschoben werden kann.

Bevorzugt ist der Autoinjektor weitergebildet, indem die Nadel eine Luer Lock Nadel ist, welche koaxial in die Aufnahme am Gehäuse mittels einem Gewinde bis an einen anschlagbildenden Boden auf einen Konus an der Spritze einschraubbar ist oder die Nadel eine Luer Lock Nadel ist, welche durch den oder im koaxialen Durchgang distal am Gehäuse auf einen Luer Lock Adapter an der Spritze anschliessbar ist oder die Nadel eine "staked needle" oder eine Kanüle ist, welche durch den koaxialen Durchgang distal am Gehäuse ragt.

Die vorliegende Erfindungs- und die Figurenbeschreibung sowie die Ansprüche beschreiben die Elemente von Teilen der erfindungsgemässen Ausführungsformen in der Regel in der grammatikalischen Einzahl. Dabei sind Ausführungsformen bei denen solche Elemente mit gleicher Struktur oder Funktion an Teilen paarweise oder mehrfach ausgeführt, insbesondere achsen- oder spiegelsymmetrisch angeordnet und/oder ausgeführt sind, immer mitgemeint bzw. mitoffenbart.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden. Es zeigen
- Fig. 1: die Komponenten einer Ausführungsform eines erfindungsgemässen Autoinjektors in perspektivischer Sicht,
- Fig. 2a,b,c: drei perspektivische Ansichten des Autoinjektors aus Fig. 1 im Zustand vor dem Gebrauch,
- Fig. 3a,b: zwei perspektivische Ansichten des Autoinjektor aus Fig. 1 im Zustand nach dem Primen,
- Fig. 4: eine perspektivische Ansicht des Autoinjektors aus Fig. 1 im Zustand nach Auslösen der Injektion,
- Fig. 5: eine perspektivische Ansicht des Autoinjektors aus Fig. 1 im Zustand nach der Injektion,
- Fig. 6a: eine proximale Aufsicht des Autoinjektors aus Fig. 1 im Zustand vor dem Gebrauch,
- Fig. 6b: eine distale Aufsicht des Autoinjektors aus Fig. 1 im Zustand vor dem Gebrauch,
- Fig. 6c: zwei Längsschnitte A-A, B-B durch den Autoinjektor aus Fig. 1 im Zustand vor dem Gebrauch,
- Fig. 6d: drei Querschnitte L-L, M-M, N-N durch den Autoinjektor aus Fig. 1 im Zustand vor dem Gebrauch,
- Fig. 7a: eine proximale Aufsicht des Autoinjektors aus Fig. 1 im Zustand während dem Primen,
- Fig. 7b: eine distale Aufsicht des Autoinjektors aus Fig. 1 im Zustand während dem Primen,
- Fig. 7c: zwei Längsschnitte C-C, D-D durch den Autoinjektor aus Fig. 1 im Zustand während dem Primen,
- Fig. 8a: eine proximale Aufsicht des Autoinjektors aus Fig. 1 im Zustand nach dem Primen,
- Fig. 8b: eine distale Aufsicht des Autoinjektors aus Fig. 1 im Zustand nach dem Primen,
- Fig. 8c: zwei Längsschnitte E-E, F-F durch den Autoinjektor aus Fig. 1 im Zustand nach dem Primen,
- Fig. 8d: einen Querschnitt P-P durch den Autoinjektor aus Fig. 1 im Zustand nach dem Primen,
- Fig. 9a: eine proximale Aufsicht des Autoinjektors aus Fig. 1 im Zustand nach Auslösen der Injektion,
- Fig. 9b: eine distale Aufsicht des Autoinjektors aus Fig. 1 im Zustand nach Auslösen der Injektion,
- Fig. 9c: zwei Längsschnitte G-G, H-H durch den Autoinjektor aus Fig. 1 im Zustand nach Auslösen der Injektion,
- Fig. 10a: eine proximale Aufsicht des Autoinjektors aus Fig. 1 im Zustand nach der Injektion,
- Fig. 10b: eine distale Aufsicht des Autoinjektors aus Fig. 1 im Zustand nach der Injektion,
- Fig. 10c: zwei Längsschnitte J-J, K-K durch den Autoinjektor aus Fig. 1 im Zustand nach der Injektion.

Der Begriff "Produkt", "Medikament" oder "medizinische Substanz" umfasst im vorliegenden Zusammenhang jede fliessfähige medizinische Formulierung, welche geeignet ist zur kontrollierten Verabreichung mittels einer Kanüle oder Hohlnadel in subkutanes oder intramuskuläres Gewebe, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Der Begriff "distal" bezeichnet eine zum vorderen, einstechseitigen Ende der Verabreichungsvorrichtung beziehungsweise zur Spitze der Injektionsnadel hin gerichtete Seite oder Richtung. Demgegenüber bezeichnet die Angabe "proximal" eine zum hinteren, dem einstechseitigen Ende gegenüberliegenden Ende der Verabreichungsvorrichtung hin gerichtete Seite oder Richtung.

Unter den Begriffen "Injektor" oder "Autoinj ektor" bzw. "Injektionssystem" werden in der vorliegenden Beschreibung Vorrichtungen verstanden, bei denen die Injektionsnadel nach erfolgter Abgabe einer kontrollierten Menge der medizinischen Substanz aus dem Gewebe entfernt wird. Somit verbleibt bei einem Injektor im Unterschied zu einem Infusionssystem die Injektionsnadel nicht über einen längeren Zeitraum von mehreren Stunden im Gewebe.

Unter den Begriffen "Priming" oder "primen" wird in der vorliegenden Beschreibung ein vorbereitender Vorgang für einen nachfolgenden therapierelevanten Injektionsvorgang bzw. die dazu erforderlichen technischen Abläufe oder Zustandsänderungen in der Vorrichtung verstanden. Es wird der Kolben in distaler Richtung im Produktbehälter verschoben was bewirkt, dass die verdrängte Flüssigkeitsmenge Luft aus dem Produktbehälter und aus dem Lumen der angeschlossenen Nadelvorrichtung mit Kanüle verdrängt, wobei ein Teil der Flüssigkeitsmenge aus dem distalen Ende der Kanüle oder Nadel in die Umgebung austreten kann. Alternativ oder zusätzlich ist damit gemeint, dass der Kolben in der Fertigspritze so weit in distaler Richtung vorgeschoben wird, dass das im Produktbehälter verbleibende verdrängbare Volumen einer bestimmten zu verabreichenden Dosis entspricht.

Unter den Begriffen "Injektion" oder "injizieren" wird in der vorliegenden Beschreibung eine therapierelevante Verabreichung, insbesondere einer bestimmten Dosis oder eines bestimmten Volumens eines Medikaments, in ein Gewebe oder Organ bzw. die dazu erforderlichen technischen Abläufe oder Zustandsänderungen in der Vorrichtung verstanden.

### FIGURENBESCHREIBUNG

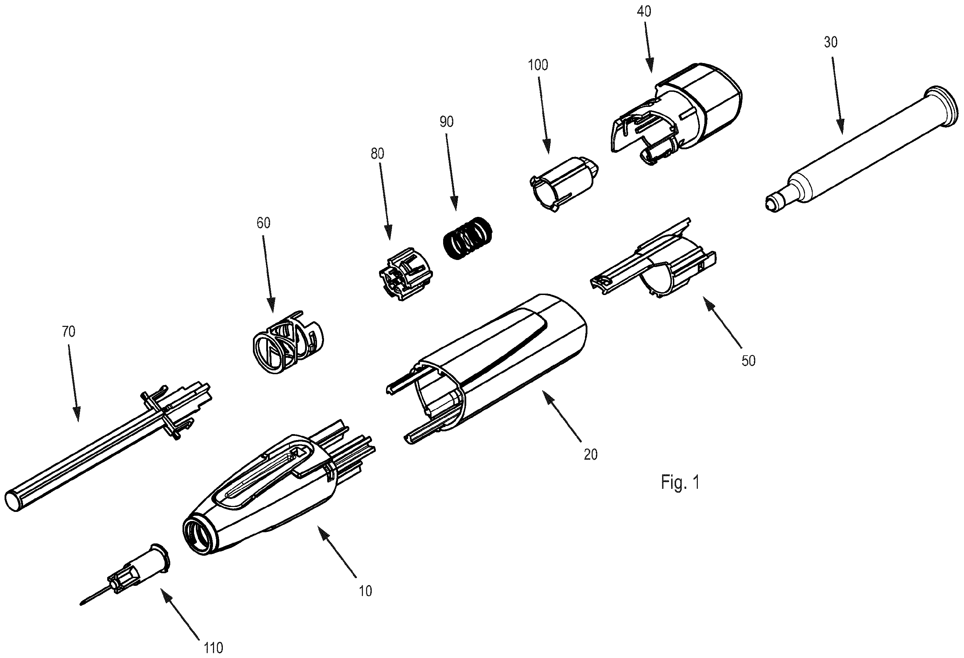

Fig. 1 zeigt die Komponenten einer beispielhaften Ausführungsform eines erfindungsgemässen Autoinjektors je in perspektivischer Sicht. Der Autoinjektor 1 weist auf: ein Frontgehäuse 10, ein Rückgehäuse 20, eine vorgefüllte Spritze 30, einen Primingknopf 40, einen Auslösering 50, einen Federring 60, eine Kolbenstange 70, einen Sperrring 80, eine Ausschüttfeder 90 und eine Signalhülse 100. Am Autoinjektor 1 ist eine Luer Lock Nadel 110 befestigt bzw. befestigbar, nachdem ggf. ein Schutzdeckel vom distalen Ende der Spritze z.B. einem Luer Konus entfernt wurde.

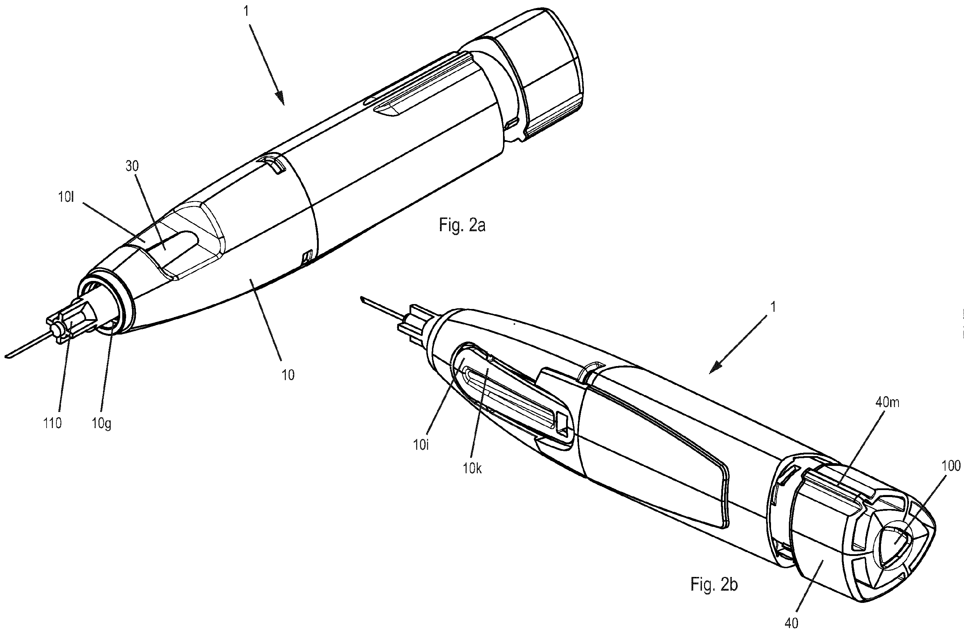

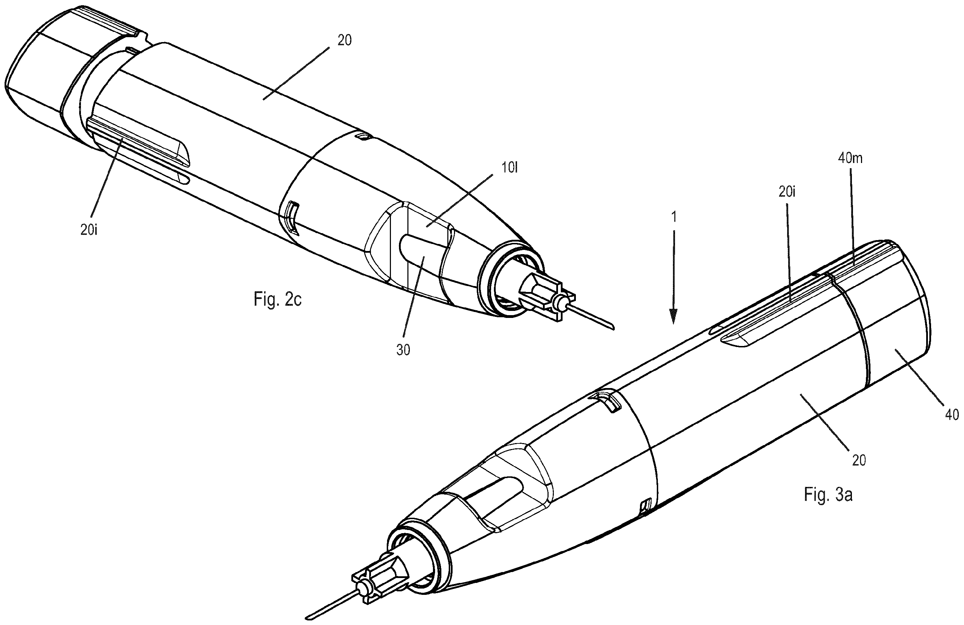

Fig. 2a,b,c zeigen drei unterschiedliche perspektivische Ansichten des Autoinjektors 1 aus Fig. 1 im Zustand vor dem Gebrauch. Fig. 2a zeigt aus distaler Sicht schräg zur Längsachse L die im Gewinde 10g befestigte Luer Lock Nadel 110 und durch ein Fenster 101 im Frontgehäuse 10 die Spritze 30. Fig. 2b zeigt aus proximaler Sicht schräg zur Längsachse L den Primingknopf 40 mit der Anzeigerippe 40m und das nicht exponierte proximale Ende der Signalhülse 100. Weiter ist das im Frontgehäuse 10 gebildete Betätigungselement als Betätigungswippe 10k mit dem Betätigungsknopf 10i sichtbar. Fig. 2c schliesslich zeigt aus distaler Sicht schräg zur Längsachse L die im proximalen Bereich des Rückgehäuses 20 gebildete Anzeigerippe 20i.

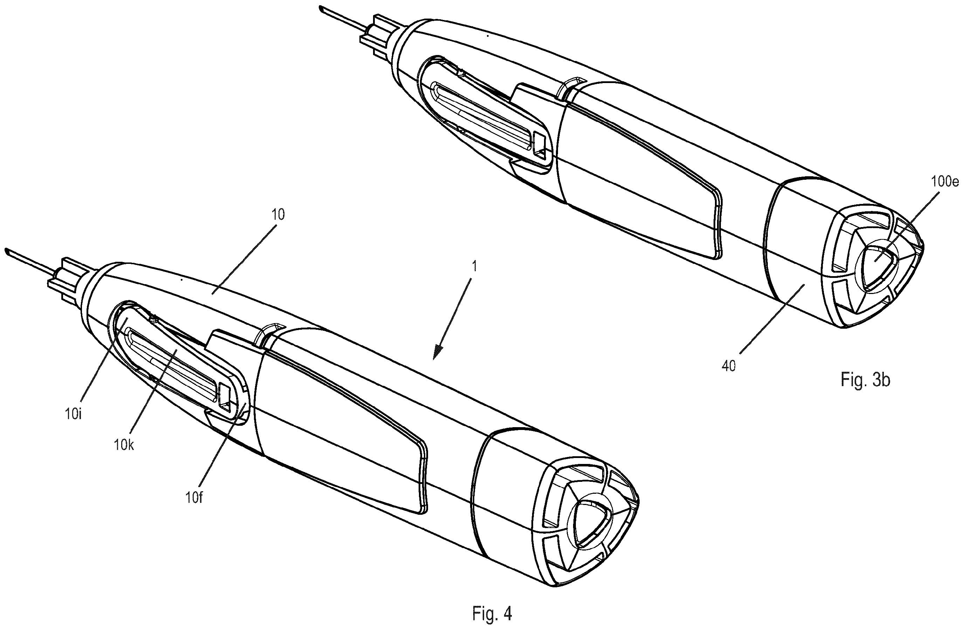

Fig. 3a,b zeigen zwei unterschiedliche perspektivische Ansichten des Autoinjektor 1 aus Fig. 1 im Zustand nach dem Primen. Fig. 3a zeigt aus distaler Sicht schräg zur Längsachse L die in Line gebrachten Anzeigerippen 20i und 40m am Rückgehäuse 20 und am Primingknopf 40. Fig. 3b zeigt aus proximaler Sicht schräg zur Längsachse L den nicht exponierten Zeiger 100e der Signalhülse 100 in der proximalen Öffnung des Primingknopfs 40.
Fig. 4 zeigt eine perspektivische Ansicht des Autoinjektors 1 aus Fig. 1 im Zustand nach Auslösen der Injektion. Am Frontgehäuse 10 ist der Betätigungsknopf 10i der Betätigungswippe 10k radial eingedrückt und daher der Auslösenocken 10f radial abgehoben.

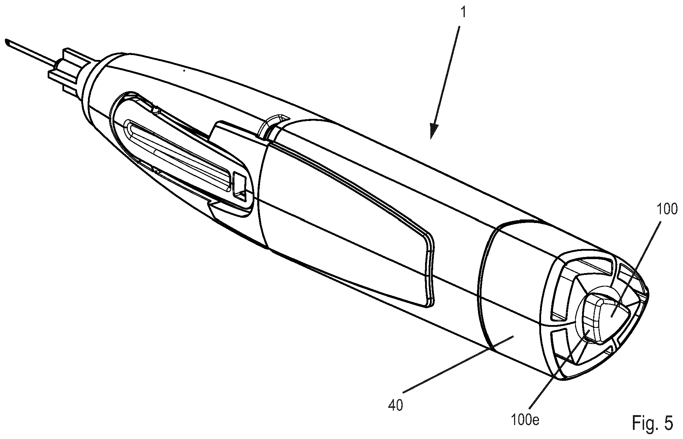

Fig. 5 zeigt eine perspektivische Ansicht des Autoinjektors 1 aus Fig. 1 im Zustand nach der Injektion. Über dem proximalen Ende des Primingknopfs 40 ist der Zeiger 100e der Signalhülse 100 exponiert sichtbar.

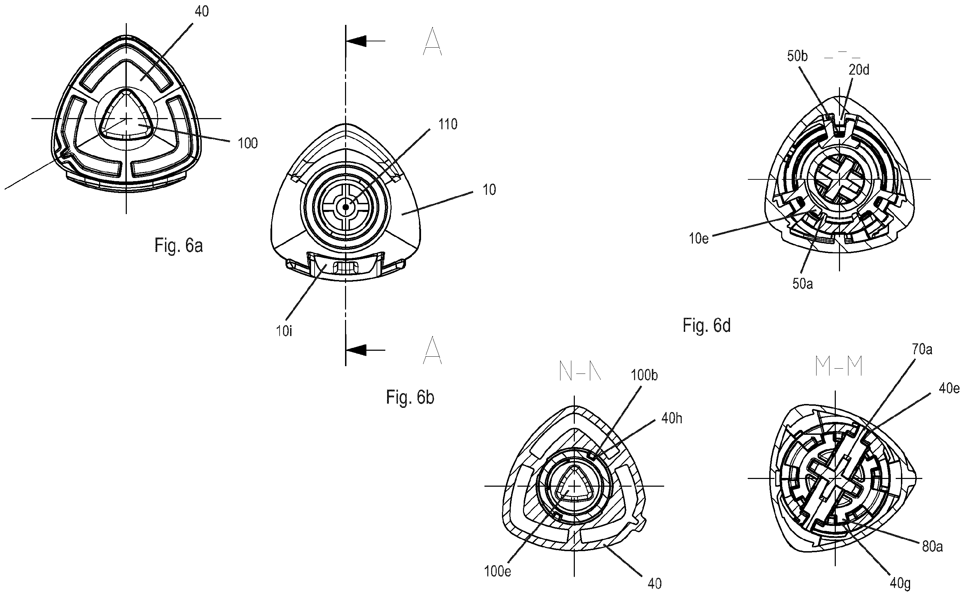

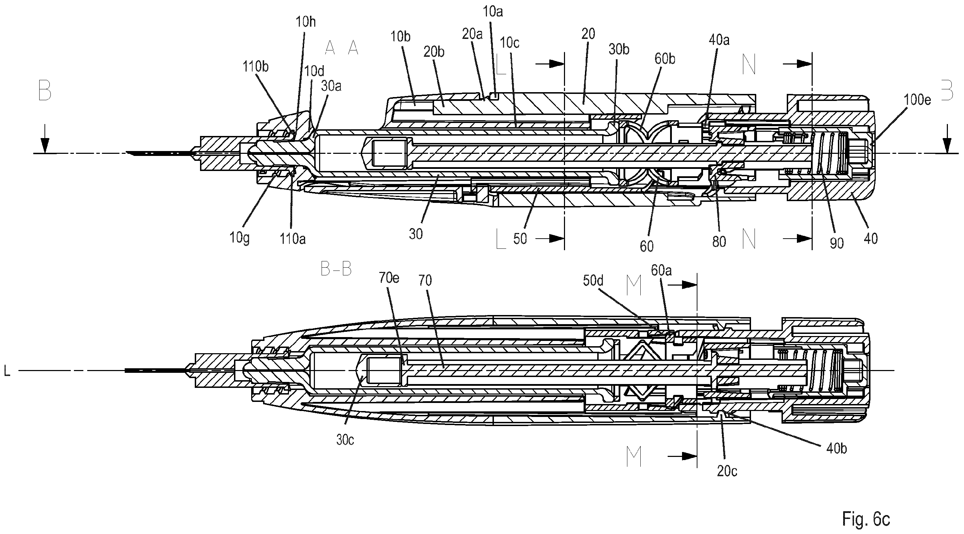

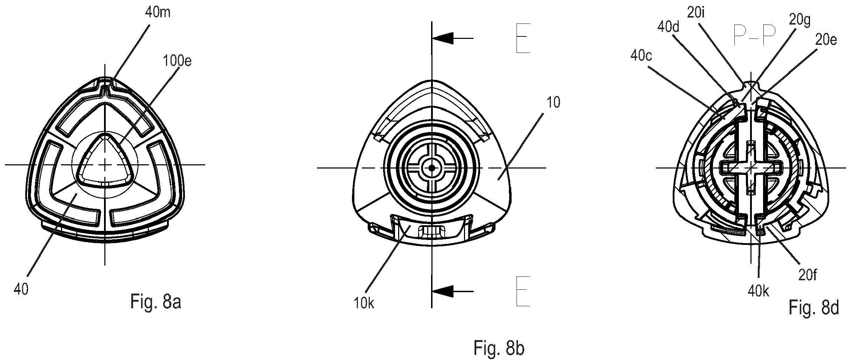

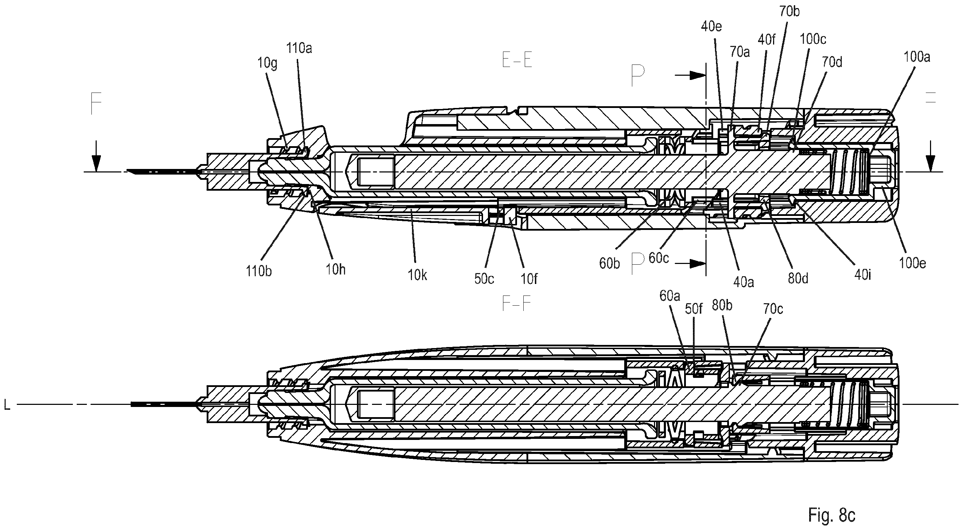

Fig. 6a zeigt eine proximale Aufsicht des Autoinjektors aus Fig. 1 im Zustand vor dem Gebrauch. Fig. 6b zeigt eine distale Aufsicht des Autoinjektors aus Fig. 1 im Zustand vor dem Gebrauch. Fig. 6c zeigt zwei Längsschnitte entlang der Längsachse L durch den Autoinjektor aus Fig. 1 im Zustand vor dem Gebrauch. Fig. 6d zeigt drei Querschnitte durch den Autoinjektor aus Fig. 1 im Zustand vor dem Gebrauch. Das Frontgehäuse 10 mit der Längsachse L kann als Bezugsteil angenommen werden, zu welchem alle übrigen Teile gemäss Fig. 1 und der weiteren Figurenbeschreibungen relativ verbunden bzw. verbindbar bzw. lösbar bzw. beweglich geführt sind und den zusammengebauten Autoinj ektors 1 bilden. Das Rückgehäuse 20 ist axial mit dem Frontgehäuse 10 verbunden indem der Schnapphaken 20a an der Schnappkante 10a einrastet. Das Rückgehäuse 20 ist zum Frontgehäuse 10 verdrehgesichert indem die Rippe 20b in die Rille 10b eingreift. Der Federring 60 hält die vorgefüllte Spritze 30 axial über den Flansch 30b in der Führung 10c des Frontgehäuses 10 wobei die Schulter 30a an der Auflage 10d anstösst. Der Auslösering 50 ist verdrehgesichert, axial verschiebbar indem die Führungsrippe 50a entlang der Rippe 10e des Frontgehäuses 10 und die Rippe 20d des Rückgehäuses 20 in der Rille 50b läuft. Der Auslösering 50 ist axial in seiner distalen Lage gehalten, indem der Auslösenocken 10f an der Betätigungswippe 10k an der Auslösekante 50c des Auslöserings 50 eingreift. Weil der Federring 60 im Zustand vor dem Gebrauch kinematisch nicht mit dem Auslösering 50 gekoppelt ist, kann der Autoinjektor 1 durch Drücken des Betätigungsknopfs 10i nicht ausgelöst werden. Der Federring 60 ist verdrehgesichert durch den Haltenocken 60a welcher axial verschiebbar in die Rille 50d des Auslöserings 50 eingreift. Der Federring 60 ist zwischen den Nocken 40a am Primingknopf 40 und dem Flansch 30b der Spritze 30 axial positioniert und wobei die Feder 60b elastisch nicht oder nur wenig vorgespannt ist. Mittels einer Rippe 20e am Rückgehäuse 20 ist der Primingknopf 40 drehbegrenzt bzw. gerastet durch den Schnapphaken 40c und den Nocken 40d (Fig. 8d). Dabei ist der Primingknopf 40 im Gewinde 40b / 20c schraubbar positioniert. Die Kolbenstange 70 ist koaxial im distalen Innern des Primingknopfs 40 gehalten. Die Stoppnocke 70a welche in der Rille 40e geführt ist bewirkt eine Verdrehsicherung und der Sperrarm 70b welcher an der Haltekante 40f eingreift bewirkt eine axiale Sicherung (Fig. 8c). Die Platte 70e am distalen Ende der Kolbenstange 70 liegt kraftfrei auf dem Kolben 30c der Spritze 30 auf oder ist von diesem durch ein kleines Spiel beabstandet. Der Sperrring 80 wird durch seine Führungsrippe 80a welche in die Führungsrille 40g des Primingknopfes 40 eingreift verdrehsicher, axial verschiebbar in diesem gehalten (Fig. 6d). Axial ist der Sperrring 80 relativ zur Kolbenstange 70 positioniert durch die flexiblen Haltearme 80b welche an der Haltekante 70c eingreifen. Die Ausschüttfeder 90 ist drehbar koaxial auf einem proximalen Abschnitt der Kolbenstange 70 gehalten. Dabei ist sie axial eingespannt zwischen dem proximalen Ende bzw. der Stirnseite der Federrippe 70d an der Kolbenstange 70 und dem Flansch 100a im Innern der Signalhülse 100. Die Kraft der gespannten Auschüttfeder 90 wird vom Sperrarm 70b und dem Schnapparm 100c in den Primingknopf 40 geleitet (Fig. 8c). Der Sperrarm 70b lagert auf der Haltekante 40f und der Schnapparm 100c auf der Haltekante 40i des Primingknopfs 40. Im Zustand vor dem Gebrauch ist der Sperrarm 70b von der Sperrippe 80d am Sperrring 80 blockiert. Der Schnapparm 100c an der Signalhülse 100 ist von der Aussenseite der Federrippe 70d blockiert. Die Signalhülse 100 ist koaxial bewegbar im Primingknopf 40 geführt. Die Führungsrippe 40h am Primingknopf 40 greift in die Führungsrille 100b der Signalhülse 100 ein und bewirkt eine relative Verdrehsicherung. Die Nadel 110 ist mit ihrem Gewinde 1 10a in das Gewinde 10g am Frontgehäuse 10 einschraubbar bis der Flansch 1 10b am Boden 10h anstösst.

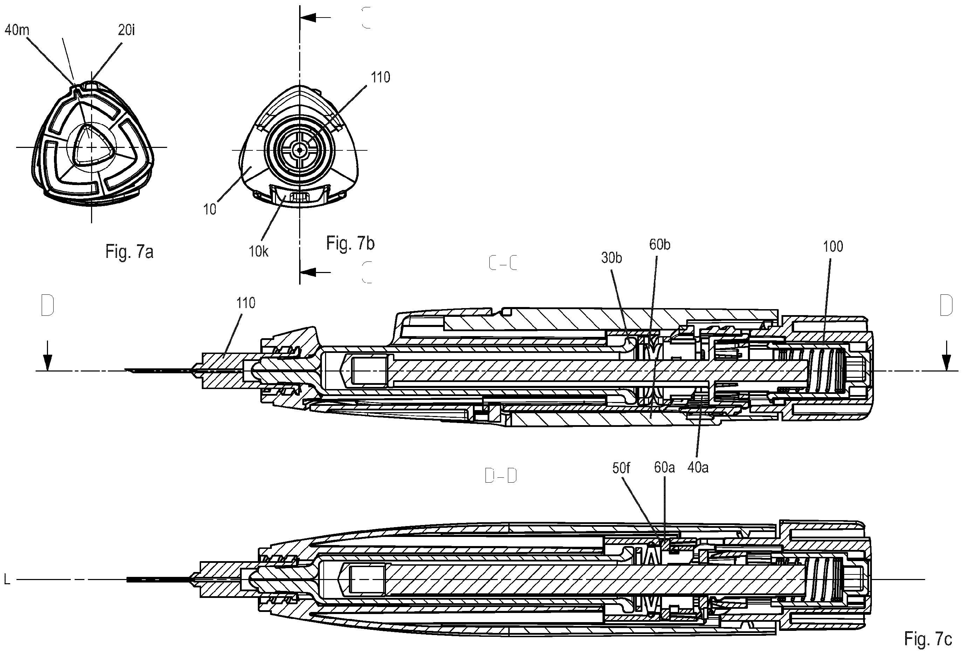

Fig. 7a zeigt eine proximale Aufsicht des Autoinjektors aus Fig. 1 im Zustand während dem Primen. Fig. 7b zeigt eine distale Aufsicht des Autoinjektors aus Fig. 1 im Zustand während dem Primen. Fig. 7c zeigt zwei Längsschnitte durch den Autoinjektor aus Fig. 1 im Zustand während dem Primen. Dabei wird der Primingknopfs 40 von Hand relativ zum Rückgehäuse 20 gedreht. Das Gewinde 40b / 20c bewirkt eine axiale Bewegung des Primingknopfs 40 wobei der mitdrehende Nocken 40a das proximale Ende des Federrings 60 in distale Richtung schiebt, wodurch dessen Feder 60b gegen den Flansch 30b der Spritze 30 gespannt bzw. weiter gespannt wird. Mit dem Primingknopf 40 bewegt sich dessen Anzeigerippe 40m in Umfangrichtung auf die Anzeigerippe 20i am Rückgehäuse 20 zu. Durch die Primingbewegung des Primingknopfs 40 in distaler Richtung werden die Haltenocken 60a am proximalen Abschnitt des Federrings 60 in der Rille 50d des Auslöserings 50 bis in die Halteöffnung 50f des Auslöserings 50 verschoben. Der Nocken 40a wird durch weiteres Vordrehen des Primingknopfs 40 durch den Ausschnitt 60c am proximalen Abschnitt des Federrings 60 freigestellt, wodurch nun die Kraft der gespannten Feder 60b in proximaler Richtung über die Haltenocken 60a und die Halteöffnung 50f auf den Auslösering 50 wirkt. Durch den Eingriff des Auslösenockens 10f an der unbetätigten Betätigungswippe 10k an der Auslösekante 50c des Auslöserings 50 ist dieser für eine proximale Bewegung in Richtung der Kraft der gespannten Feder 60b gesperrt (Fig. 8c). Der Nocken 40d am Schnapphaken 40c federt zum Ende der Primingbewegung radial aus und schlägt auf den Nocken 20g am Rückgehäuse 20 (Fig. 8d) und signalisiert mit einem Klickgeräusch das Ende der Primingbewegung. Durch die mittels dem Sperrring 80 fixierte Verbindung der Kolbenstange 70 mit dem Primingknopf 40 verschiebt sich die Kolbenstange 70 relativ zur Spritze 30 und bewirkt durch Vorschieben des Kolbens 30c in der Spritze 30 das Primen des Autoinjektors 1.
Fig. 8a zeigt eine proximale Aufsicht des Autoinjektors aus Fig. 1 im Zustand nach dem Primen. Fig. 8b zeigt eine distale Aufsicht des Autoinjektors aus Fig. 1 im Zustand nach dem Primen. Fig. 8c zeigt zwei Längsschnitte durch den Autoinjektor aus Fig. 1 im Zustand nach dem Primen. Fig. 8d zeigt einen Querschnitt durch den Autoinjektor aus Fig. 1 im Zustand nach dem Primen. Der Schnapphaken 40c ist eingehängt im Rückgehäuse 20 und verhindert eine Rückdrehen des Primingknopfs 40. Der Anschlagnocken 40k am Primingknopf 40 stösst an der Anschlagrippe 20f an und verhindert ein weiteres Vordrehen des Primingknopfs 40. Der Zustand nach dem Primen ist ersichtlich durch die axiale Alinierung der Anzeigerippe 20i am Rückgehäuse 20 mit der Anzeigerippe 40m am Primingknopf.

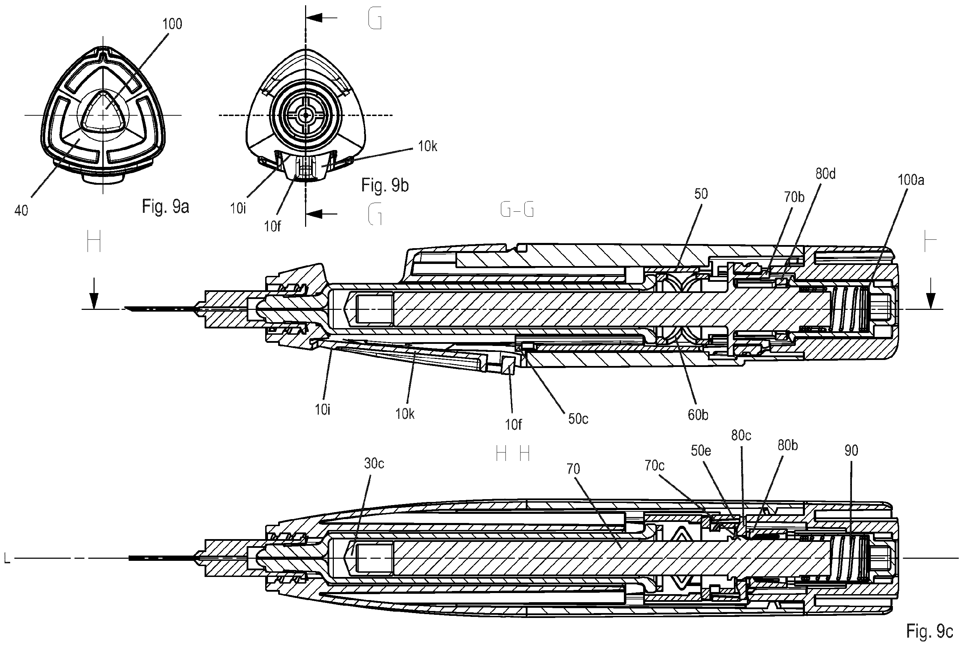

Fig. 9a zeigt eine proximale Aufsicht des Autoinjektors aus Fig. 1 im Zustand nach Auslösen der Injektion. Fig. 9b zeigt eine distale Aufsicht des Autoinjektors aus Fig. 1 im Zustand nach Auslösen der Injektion. Fig. 9c zeigt zwei Längsschnitte durch den Autoinjektor aus Fig. 1 im Zustand nach Auslösen der Injektion. Die Betätigungswippe 10k ist mit einem Filmgelenk einstückig mit dem Frontgehäuse 10 gebildet. Bei radialem Eindrücken bzw. Betätigen des Betätigungsknopfs 10i verdreht sich das Filmgelenk der Betätigungswippe 10k und der Auslösenocken 10f wird aus dem Eingriff mit der Auslösekante 50c des Auslöserings 50 gehoben. Die Kraft der Feder 60b schiebt den Auslösering 50 in proximale Richtung. Mittels der proximalen Kante 50e welche den distalen Nocken 80c des Sperrrings 80 stösst wird dieser in proximale Richtung mitverschoben. Dabei löst sich der flexible Haltearm 80b aus dem Eingriff mit der Haltekante 70c an der Kolbenstange 70. Die Sperrrippe 80d am Sperrring 80 wird in axialer Richtung vom Sperrarm 70b entfernt und stellt diesen frei für eine radiale Auslenkung zur Längsachse L hin. Diese Auslenkung des Sperrarms 70b wird unmittelbar bewirkt durch die Kraft der Ausschüttfeder 90 welche in distaler Richtung über das proximale Ende der Federrippe 70d auf die Kolbenstange 70 wirkt und diese in distale Richtung relativ zur Spritze 30 verschiebt und bewirkt durch Vorschieben des Kolbens 30c in der Spritze 30 das Ausschütten von Medikament aus dem Autoinjektors 1.

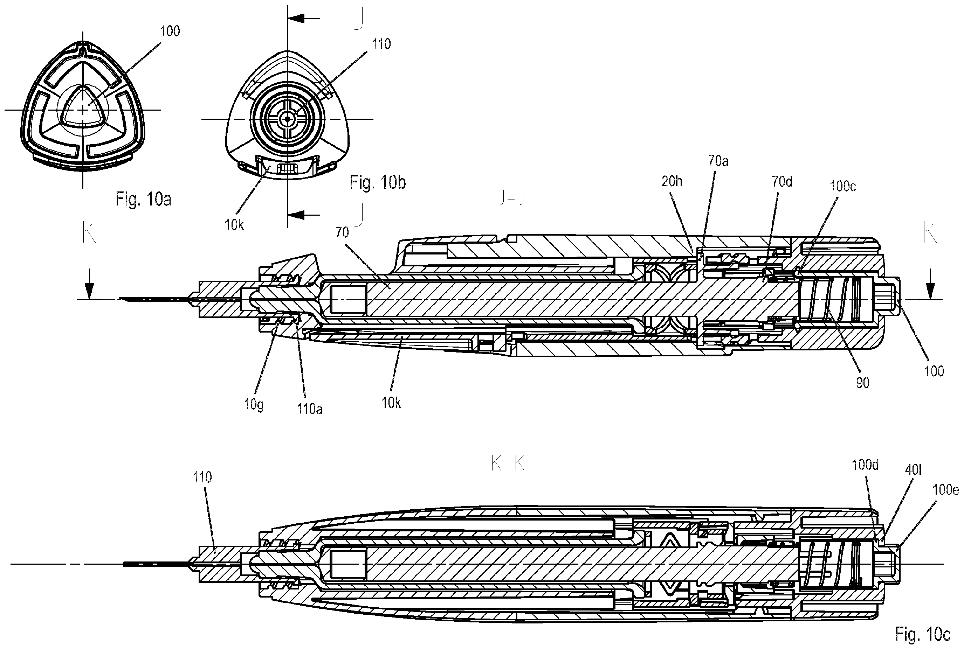

Fig. 10a zeigt eine proximale Aufsicht des Autoinjektors aus Fig. 1 im Zustand nach der Injektion. Fig. 10b zeigt eine distale Aufsicht des Autoinjektors aus Fig. 1 im Zustand nach der Injektion. Fig. 10c zeigt zwei Längsschnitte durch den Autoinjektor aus Fig. 1 im Zustand nach der Injektion. Kurz vor Ende der Verschiebung der Kolbenstange 70 entfernt sich die Federrippe 70d axial vom Schnapparm 100c der Signalhülse 100 stellt diesen frei für eine radiale Auslenkung zur Längsachse L hin. Diese Auslenkung des Schnapparms 100c wird unmittelbar bewirkt durch die Kraft der Ausschüttfeder 90 welche in proximaler Richtung über den Flansch 100a auf die Signalhülse 100 wirkt und diese in proximale Richtung relativ zum Primingknopf 40 schiebt bis der Anschlagflansch 100d auf den Anschlag 40l am Primingknopf 40 auftrifft und dabei hörbar und spürbar das Ende der Injektion mit einem Klickgeräusch signalisiert. Der Zeiger 100e signalisiert das Ende der Injektion andauernd, indem er durch die Verschiebung der Signalhülse 100 über das proximalen Ende des Primingknopfs 40 axial abragt. Die Kolbenstange 70 und damit auch die Ausschüttung von Medikament kommt durch Anschlagen des Stoppnockens 70a an der Stoppkante 20h des Rückgehäuses 20 zum Stillstand. Damit ist die Präzision der Ausschüttmenge bzw. der Kolbenstangenhub weitgehend durch die Kunststoffteile des Autoinjektors 1 bestimmt und Geräten aus dem Stand überlegen, bei welchen die Ausschüttung durch Blockieren des Kolbens am Schulterbereich der Spritze ein Ende findet.

Die Nadel 110 kann nach dem Gebrauch des Autoinjektors 1 von diesem getrennt und sicher entsorgt werden.

### BEZUGSZEICHENLISTE

| | | | |
|---|---|---|---|
| 1 | Autoinjektor | 50 | Auslösering, Auslöseelement |
| 10 | Frontgehäuse | 50a | Führungsrippe |
| 10a | Schnappkante | 50b | Rille |
| 10b | Rille | 50c | Auslösekante |
| 10c | Führung | 50d | Rille |
| 10d | Auflage | 50e | Kante |
| 10e | Rippe | 50f | Halteöffnung |
| 10f | Auslösenocken | 60 | Federring, zweite Feder |
| 10g | Gewinde | 60a | Haltenocken |
| 10h | Boden | 60b | Feder |
| 10i | Betätigungsknopf | 60c | Ausschnitt |
| 10k | Betätigungswippe,-element | 70 | Kolbenstange |
| 10l | Fenster | 70a | Stoppnocken |
| 20 | Rückgehäuse | 70b | Sperrarm |
| 20a | Schnapphaken | 70c | Haltekante |
| 20b | Rippe | 70d | Federrippe |
| 20c | Gewinde | 70e | Platte |
| 20d | Rippe | 80 | Sperrring |
| 20e | Rippe | 80a | Führungsrippe |
| 20f | Anschlagrippe | 80b | Haltearme |
| 20g | Nocken | 80c | Nocken |
| 20h | Stoppkante | 80d | Sperrrippe |
| 20i | Anzeigerippe | 90 | Ausschüttfeder, erste Feder |
| 30 | Spritze | 100 | Signalhülse |
| 30a | Schulter | 100a | Flansch |
| 30b | Flansch | 100b | Führungsrille |
| 30c | Kolben | 100c | Schnapparm |
| 40 | Knopf, Primingknopf | 100d | Anschlagflansch |
| 40a | Nocken | 100e | Zeiger |
| 40b | Gewinde | 110 | Nadel |
| 40c | Schnapphaken | 110a | Gewinde |
| 40d | Nocken | 110b | Flansch |
| 40e | Rille | L | Längsachse |
| 40f | Haltekante | | |
| 40g | Führungsrille | | |
| 40h | Führungsrippe | | |
| 40i | Haltekante | | |
| 40k | Anschlagnocken | | |
| 40l | Anschlag | | |
| 40m | Anzeigerippe | | |

## Patentansprüche

1. Autoinjektor (1) zur Verabreichung eines Medikaments umfassend:
- ein einteiliges oder mehrteiliges Gehäuse (10,20) mit einer Längsachse (L) und einer gehäusefest koaxial aufgenommenen Spritze (30) mit einem verschiebbaren Kolben (30c)
- eine distal am Gehäuse (10,20) angebrachte Aufnahme (10g,10h) oder einen koaxialen Durchgang für eine Nadel (110)
- einen proximal am Gehäuse (10,20) angebrachten von Hand schraubbaren Knopf (40)
- eine axial wirkende erste Feder (90) welche vorgespannt im Knopf (40) koaxial gelagert ist
- eine am Knopf (40) befestigte und von diesem lösbare Kolbenstange (70) welche von der ersten Feder (90) in distaler Richtung kraftbeaufschlagt ist
- ein Auslöseelement (50) welches in einer ersten Lage die Kolbenstange (70) axial gegen die Kraft der ersten Feder (90) zum schraubbaren Knopf (40) befestigt hält und welches Auslöseelement (50) in einer zweiten Lage die Kolbenstange (70) vom Knopf (40) lösbar macht wodurch der verschiebbare Kolben (30c) in der Spritze (30) von der Kraft der ersten Feder (90) mittels der gelösten Kolbenstange (70) in distale Richtung um einen Kolbenweg verschoben werden kann **dadurch gekennzeichnet, dass**
das Auslöseelement (50) für eine Bewegung in die zweite Lage kraftbeaufschlagbar wird, während der Knopf (40) und die daran befestigte Kolbenstange (70) aus einer axialen Anfangsposition relativ zum Gehäuse (10,20) durch Einschrauben ins Gehäuse (10,20) eine axiale Primingposition erreichen, wobei die Kolbenstange (70) den Kolben (30c) in der Spritze (30) in distale Richtung stösst.

2. Autoinjektor (1) nach dem vorhergehenden Anspruch, weiter aufweisend:
- eine axial wirkende zweite Feder (60) welche mit einem distalen Ende am proximalen Flansch (30b) der Spritze (30) und mit einem proximalen Ende an einem distalen Nocken (40a) am Knopf (40) axial gelagert ist **dadurch gekennzeichnet, dass**
ein elastischer Abschnitt (60b) der zweiten Feder (60) durch das Einschrauben des Knopfs (40) ins Gehäuse (10,20) gespannt wird und vor dem Erreichen der axialen Primingposition das proximale Ende der zweiten Feder (60) vom distalen Nocken (40a) am Knopf (40) kinematisch auf das Auslöseelement (50) wechselt und dieses kraftbeaufschlagt indem das proximale Ende der zweiten Feder (60) das Auslöseelement (50) in proximale Richtung drängt, insbesondere indem durch das Einschrauben des Knopfs (40) in das Gehäuse (10,20) ein Haltenocken (60a) der zweiten Feder (60) in eine Halteöffnung (50f) einer Rille (50d) des Auslöseelements (50) einkuppelt und der distale Nocken (40a) am Knopf (40) durch einen axialen Ausschnitt am Endabschitt freigestellt wird.

3. Autoinjektor (1) nach dem vorhergehenden Anspruch,
**dadurch gekennzeichnet, dass**
der elastischer Abschnitt (60b) der zweiten Feder (60) aus einem Kunststoff gebildet ist.

4. Autoinjektor (1) nach einem der vorhergehenden Ansprüche,
**dadurch gekennzeichnet, dass**
der schraubbare Knopf (40) und das Gehäuse (10,20) am äusseren Umfang je eine Markierung (20i,40m) aufweisen, welche in der axialen Primingposition des Knopfs (40) aufeinander weisen, insbesondere in achsparalleler Linie zu stehen kommen.

5. Autoinjektor (1) nach einem der vorhergehenden Ansprüche,
**dadurch gekennzeichnet, dass**
der schraubbare Knopf (40) einen Schnapphaken (40c) aufweist, welcher beim Erreichen der axialen Primingposition radial nach aussen federt und sich am Gehäuse (10,20) festhängt.

6. Autoinjektor (1) nach einem der vorhergehenden Ansprüche, weiter aufweisend:
- einen proximal vom Auslöseelement (50) gebildeten und von diesem in proximale Richtung bewegbaren Sperrring (80)
**dadurch gekennzeichnet, dass**
der Sperrring (80) in der ersten Lage des Auslöseelements (50) einen an der Kolbenstange (70) gebildeten Sperrarm (70b) am radialen Auslenken hindert und im Eingriff mit einer Haltekante (40i) am Knopf (40) hält und/oder der Sperrring (80) in der zweiten Lage des Auslöseelements (50) den an der Kolbenstange (70) gebildeten Sperrarm (70b) für eine radiale Auslenkung freigibt, wodurch die Kolbenstange (70) vom Knopf (40) lösbar wird.

7. Autoinjektor (1) nach einem der vorhergehenden Ansprüche, weiter aufweisend:
- ein insbesondere seitlich am Gehäuse (10,20) gebildetes Betätigungselement (10k)
**dadurch gekennzeichnet, dass**
das Betätigungselement (10k) in einer unbetätigten Position das Auslöseelement (50) in der ersten Lage hält und/oder das Betätigungselement (10k) in seiner betätigten Position das insbesondere kraftbeaufschlagte Auslöseelement (50) für eine Bewegung in die zweite Lage freigibt.

8. Autoinjektor (1) nach einem der vorhergehenden Ansprüche, wobei der Kolben (30c) von der Kraft der ersten Feder (90) mittels der gelösten Kolbenstange (70) in distale Richtung um den Kolbenweg verschiebbar ist an dessen Ende die Kolbenstange (70) mit einem Stoppnocken (70a) an einer Stoppkante (20h) am Gehäuse (10,20) auftrifft
**dadurch gekennzeichnet, dass**
das durch den Kolbenweg aus der Spritze (30) verdrängbare Volumen aus dem Bereich 0.01 bis 0.5 ml, bevorzugter 0.02 bis 0.1 ml, noch bevorzugter 0.04 bis 0.06 ml, insbesondere 0.05 ml ist.

9. Autoinjektor (1) nach einem der vorhergehenden Ansprüche, weiter aufweisend:
- eine koaxial im Knopf (40) gelagerte durch die proximal wirkende Kraft der ersten Feder (90) um einen Hub aus einer Ausgangslage verschiebbaren Signalhülse (100)
**dadurch gekennzeichnet, dass**
die Signalhülse am Ende des Hubs mittels eines Anschlagflansches (100d) auf einen Anschlag (40l) am Knopf (40) auftrifft und/oder ein verjüngter Endabschnitt (100e) der Signalhülse (100) am proximalen Ende des Knopfes (40) exponiert wird.

10. Autoinjektor (1) nach dem vorhergehenden Anspruch,
**dadurch gekennzeichnet, dass**
die Signalhülse (100) in der Ausgangslage über einen durch Auslenkung lösbaren Schnapparm (100c) an einer Haltekante (40i) am Knopf (40) befestigt ist, wobei der Schnapparm (100c) von der Aussenseite einer Rippe (70d) an der Kolbenstange (70) bis vor dem Ende des Kolbenhubs an der Auslenkung gehindert wird.

11. Autoinjektor (1) nach dem vorhergehenden Anspruch,
**dadurch gekennzeichnet, dass**
die proximale Stirnseite der Rippe (70d) an der Kolbenstange (70) das distale Ende der ersten Feder (90) lagert und/oder der Flansch (100a) der Signalhülse (100) das proximale Ende der ersten Feder (90) lagert.

12. Autoinjektor (1) nach einem der vorhergehenden Ansprüche,
**dadurch gekennzeichnet, dass**
die Nadel (110) eine Luer Lock Nadel ist, welche koaxial in die Aufnahme (10g,10h) am Gehäuse (10,20) mittels einem Gewinde (10g) bis an einen anschlagbildenden Boden (10h) auf einen Konus an der Spritze (30) einschraubbar ist oder die Nadel (110) eine Luer Lock Nadel ist, welche durch den koaxialen Durchgang distal am Gehäuse (10,20) auf einen Luer Lock Adapter an der Spritze (30) anschliessbar ist oder die Nadel (110) eine "staked needle" ist welche durch den koaxialen Durchgang distal am Gehäuse (10,20) ragt.
